# EUROPEAN PATENT APPLICATION

(11) **EP 4 506 359 A1**
(43) Date of publication of application: **12.02.2025**
(21) Application number: 22923474.5
(22) Date of filing: 02.12.2022
(51) Int. Cl.: C07K 14/725, C12N 15/12, C12N 15/63, A61K 38/17, A61P 31/22

(54) **TCR TARGETING CYTOMEGALOVIRUS ANTIGEN, T CELL EXPRESSING TCR, AND APPLICATION**

(30) Priority: 27.01.2022 CN 202210099611
(71) Applicant: Shanghai General Hospital, Shanghai 200080 (CN)
(72) Inventor: WANG, Pengran, Shanghai 200080 (CN); SHEN, Lianghua, Shanghai 200080 (CN); ZHANG, Yan, Shanghai 200080 (CN); SONG, Xianmin, Shanghai 200080 (CN)
(74) Representative: Diehl & Partner
(86) International application number: PCT/CN2022/136297
(87) International publication number: WO 2023/142683

(57) **Abstract**

The present invention provides a T cell receptor (TCR) targeting a cytomegalovirus (CMV) antigen, a T cell expressing the TCR, and an application. Specitically, disclosed in the present invention are a specific T cell for HLA-A*0201-restricted targeting of a CMV-pp65 antigenic epitope and an application thereof. The TCR carried by the T cell can specifically target CMV-pp65, and clear the CMV precisely and quickly.

## Description

### Technical field

The invention relates to a TCR capable of recognizing cytomegalovirus antigen peptides, a cytomegalovirus specific T cell expressing the TCR, and their use in preventing and treating cytomegalovirus related diseases.

### Background

Cytomegalovirus (CMV) belongs to the beta subfamily of the family Herpesviridae, which has obvious host species specificity. It is the largest and most complex virus in the family Herpesviridae; CMV infection is very widespread in the population. The adult infection rate is more than 95%. It is usually recessive infection. Most infected people have no clinical symptoms, but under certain conditions, it can invade multiple organs and systems and cause serious diseases. In recent years, great progress has been made in the diagnosis and treatment of CMV infection, which has significantly reduced the incidence of CMV infection after transplantation, but the incidence of CMV infection in allogeneic stem cell transplantation (allo HSCT) patients remains high, and its indirect effects, such as secondary implantation dysfunction, GVHD, all-cause mortality, still have a huge impact on the prognosis of patients. However, the existing anti-CMV drugs also have many shortcomings, such as single dosage form, similar target, and can lead to granulocyte deficiency/kidney damage. Current antiviral drugs, such as acyclovir and ganciclovir, often cause serious side effects, such as bone marrow suppression.

Human cytomegalovirus (HCMV) contains about 20-25 proteins, among which three proteins play the most important role in mediating HCMV infection and virus replication, namely pp65 protein encoded by ORF UL83, phosphoprotein PP71 encoded by ORF UL82, and pUL69 protein encoded by ORF UL69. The common feature of the above three proteins is to initiate the infection of HCMV on host cells and viral replication, and to play an important role in escaping T lymphocyte mediated cytotoxicity. Studies have shown that among the above three proteins, pp65 protein has the most serious effect on graft vasculopathy and rejection. The immune impairment caused by allo-HSCT will reactivate the latent HCMV, leading to serious clinical complications.

In recent years, cellular immunotherapy represented by immune checkpoint inhibitors, CAR-T (Chimeric Antibody Receptor Engineered T cell) and TCR-T (T cell Receptor Engineered T Cell) has made significant progress in the field of tumor treatment. TCR-T therapy is to capture the specific TCR against tumor antigens or specific viral antigens, and to modify T cells by genetic engineering technology, which can achieve the purpose of treating tumors or clearing viral infections after infusion into the body.

Patients with allo-HSCT are prone to primary CMV infection or latent CMV reactivation due to their low immune function and inability to effectively clear CMV. After the occurrence of CMV infection/reactivation in HSCT patients, it can cause a series of related diseases from CMV related fever to organ involvement, which is closely related to disease recurrence and subject survival, and the current conventional drug treatment effect is poor. Therefore, if CMV-TCR-T cell therapy can be applied to the treatment and prevention of CMV infection after HSCT or other organ transplantation, it has great clinical value and application prospects.

### Summary of invention

The object of the present invention is to provide a specific TCR of HLA-A*0201-restricted targeting of cytomegalovirus antigenic epitope pp65, a TCR-T cell expressing the TCR and its application.

The first aspect of the present invention provides a T cell receptor (TCR), which comprises a TCRα chain variable domain and a TCRβ chain variable domain, wherein the amino acid sequence of CDR3 of the TCRα chain variable domain is CAFPYNNNDMRF (SEQ ID No. 13); and/or

The amino acid sequence of CDR3 of the TCRβ chain variable domain is CASSLEGYTEAFF (SEQ ID No. 21).

In another preferred example, the TCR specifically binds to NLVPMVATV-HLA-A*0201 complex.

In another preferred example, the three complementarity determining regions (CDRs) of the TCRα chain variable domain are:
α-CDR1: SSNFYA (SEQ ID NO.9),
α-CDR2: MTLNGDE (SEQ ID NO.11),
α-CDR3: CAFPYNNNDMRF (SEQ ID NO.13).

In another preferred example, the three complementarity determining regions of the TCRβ chain variable domain are:
β-CDR 1: MNHEY (SEQ ID NO.17),
β-CDR 2: SMNVEV (SEQ ID NO.19),
β-CDR 3: CASSLEGYTEAFF (SEQ ID NO.21).

In another preferred example, the TCR comprises a TCRα chain variable domain and a TCRβ chain variable domain, wherein the TCRα chain variable domain is an amino acid sequence with at least 90% sequence identity with SEQ ID NO.7; and/or the TCRβ chain variable domain is an amino acid sequence with at least 90% sequence identity with SEQ ID NO.15.

In another preferred example, the TCR comprises the α chain variable domain amino acid sequence of SEQ ID NO.7.

In another preferred example, the TCR comprises the β chain variable domain amino acid sequence of SEQ ID NO.15.

In another preferred example, the TCR is an αβ heterodimer, which comprises the TCRα chain constant region TRAC*01 and the TCRβ chain constant region TRBC1*01 or TRBC2*01.

In another preferred example, the α chain amino acid sequence of the TCR is SEQ ID NO.3.

In another preferred example, the β chain amino acid sequence of the TCR is SEQ ID NO.5.

In another preferred example, the α chain and β chain of the TCR contain an artificial interchain disulfide bond.

In another preferred example. the amino acid sequence of the TCR is set forth as SEQ ID NO.1.

The second aspect of the present invention provides a multivalent TCR complex, which comprises at least two TCR molecules, and at least one of the TCR molecules is the TCR described in the first aspect of the present invention.

The third aspect of the present invention provides a nucleic acid molecule, which comprises a nucleic acid sequence encoding the TCR molecule described in the first aspect of the present invention or a complementary sequence thereof.

In another preferred example, the nucleic acid molecule comprises a nucleotide sequence of SEQ ID NO.8 encoding the variable domain of TCRα chain.

In another preferred example, the nucleic acid molecule comprises a nucleotide sequence of SEQ ID NO.16 encoding the variable domain of TCRβ chain.

In another preferred example, the nucleic acid molecule comprises a nucleotide sequence of SEQ ID NO.4 encoding the TCRα chain.

In another preferred example, the nucleic acid molecule comprises a nucleotide sequence of SEQ ID NO.6 encoding the TCRβ chain.

In another preferred example, the nucleic acid molecule comprises a nucleotide sequence of SEQ ID NO.2.

The fourth aspect of the present invention provides a vector containing the nucleic acid molecule described in the third aspect of the present invention; preferably, the vector is a viral vector; more preferably, the vector is a lentiviral vector.

The fifth aspect of the present invention provides an isolated host cell, which contains the vector described in the fourth aspect of the present invention or is integrated with the exogenous nucleic acid molecule described in the third aspect of the present invention in its genome.

The sixth aspect of the present invention provides a cell that is transduced with the nucleic acid molecule described in the third aspect of the present invention or the vector described in the fourth aspect of the present invention; preferably, the cell is a T cell or stem cell.

The seventh aspect of the invention provides a pharmaceutical composition, which contains a pharmaceutically acceptable carrier and the TCR described in the first aspect of the present invention, the TCR complex described in the second aspect of the present invention, the nucleic acid molecule described in the third aspect of the present invention, the vector described in the fourth aspect of the present invention, or the cell described in the sixth aspect of the present invention.

The eighth aspect of the present invention provides use of the T cell receptor described in the first aspect of the present invention, or the TCR complex described in the second aspect of the present invention, the nucleic acid molecule described in the third aspect of the present invention, the vector described in the fourth aspect of the present invention, or the cell described in the sixth aspect of the present invention, in the manufacture of drugs for treating cytomegalovirus related diseases.

The ninth aspect of the present invention provides a method for treating diseases, comprising administering an appropriate amount of the T cell receptor described in the first aspect of the present invention, or the TCR complex described in the second aspect of the present invention, the nucleic acid molecule described in the third aspect of the present invention, the vector described in the fourth aspect of the present invention, or the cell described in the sixth aspect of the present invention, or the pharmaceutical composition described in the seventh aspect of the present invention to a subject in need of treatment;

Preferably, the disease is cytomegalovirus infection related disease (cytomegalovirus infection), such as CMV retinitis, CMV pneumonia, CMV gastroenteritis, CMV encephalitis, etc.

It should be understood that within the scope of the present invention, the above technical features of the present invention and the technical features specifically described below (as those in examples) can be combined with each other to form a new or preferred technical solution. Due to space limitations, they will not be repeated herein.

### Description of drawings

FIG. 1: Specific stimulation and screening process of CMV-pp65-TCR-T;
FIG. 2: specific expansion results of CMV-pp65-TCR-T cells in vitro;
FIG. 3: information of CMV-pp65-TCR-T vector;
FIG. 4: in vitro binding validation of CMV-pp65-TCR-T;
FIG. 5: validation of CMV-pp65-TCR-T activation in vitro;
FIG. 6: in vitro killing validation of CMV-pp65-TCR-T;
FIG. 7: subcutaneous tumor implantation model for in vivo killing validation of CMV-pp65-TCR-T;
FIG. 8: metastasis model for in vivo killing validation of CMV-pp65-TCR-T.

### Detailed description

The present invention discloses a specific T cell for HLA-A*0201-restricted targeting of a CMV-pp65 antigenic epitope NLVPMVATV and an application thereof, the T cell receptor (TCR) carried by the T cell can specifically target CMV-pp65 and clear CMV virus accurately and rapidly. CMV-pp65 specific TCR-T cells can be prepared after lentiviral infection of T cells with CMV-pp65 high affinity TCR gene. In one aspect, CMV-pp65-TCR-T cells can rapidly clear CMV virus through precise targeting; in another aspect, it can also play a long-term protective role through TCR-T cell-mediated immune reconstitution, the thorough clearance of virus and prevention of reinfection indirectly. Therefore, the pp65-TCR-T cells specific for the pp65 antigenic epitope of the present invention can specifically bind to and kill the target cells of HLA-A*0201 restricted expression of CMV pp65 antigen, and can provide a new clinical scheme for TCR-T treatment of CMV related diseases. It is of great clinical value and application prospect to treat and prevent CMV infection after HSCT or other organ transplantation.

### Definition

MHC molecules are proteins of the immunoglobulin superfamily, which can be MHC class I or class II molecules. Therefore, it is specific for antigen presentation. Different individuals have different MHCs, which can present different short peptides of a protein antigen to their respective APC cell surface. Human MHC is often referred to as HLA gene or HLA complex.

T cell receptor (TCR) is the only receptor for specific antigenic peptides presented on the major histocompatibility complex (MHC). In the immune system, the combination of antigen-specific TCR and pMHC complex triggers direct physical contact between T cells and antigen-presenting cells (APCs), and then the interaction between other cell membrane surface molecules of T cells and APCs, which causes a series of subsequent cell signaling and other physiological reactions, so that T cells with different antigen specificities can exert immune effects on their target cells.

TCR is a glycoprotein on the surface of cell membrane, which is composed of α chain/β chain or y chain/δ chain in the form of heterodimer. TCR heterodimers consist of α and β chains in 95% of T cells, while 5% of T cells have TCRs consisting of γ and δ chains. Natural αβ heterodimeric TCRs have α chains and β chains, which constitute subunits of αβ heterodimeric TCRs. In a broad sense, each chain of α and β contains a variable region, a connecting region and a constant region. The β chain usually also contains a short polytropic region between the variable region and the connecting region, but the polytropic region is often regarded as a part of the connecting region. Each variable region contains three CDRs (complementarity determining regions), CDR1, CDR2, and CDR3, which are chimeric in the framework regions. The CDR regions determine the binding of TCR to the pMHC complex, wherein CDR3 is recombined from the variable region and the connecting region, known as the hypervariable region. The α and β chains of TCR are generally regarded as having two "domains" respectively, i.e. variable domain and constant domain. The variable domain is composed of a connected variable region and a connected region. The sequence of TCR constant domain can be found in the public database of the international ImMunoGeneTics information system (IMGT), for example, the constant domain sequence of TCR α chain is "TRAC*01", and the constant domain sequence of TCR β chain is "TRBC1*01" or "TRBC2*01". In addition, the α and β chains of TCR also contain transmembrane regions and cytoplasmic regions.

In the present invention, the terms "polypeptide of the present invention", "TCR of the present invention" and "T cell receptor of the present invention" are used interchangeably.

### Natural interchain disulfide bond and artificial interchain disulfide bond

There is a group of disulfide bonds between Cα and Cβ chains in the membrane-proximal region of natural TCR, which is called "natural interchain disulfide bond" in the present invention. In the present invention, the interchain covalent disulfide bond that is artificially introduced and whose position is different from that of the natural interchain disulfide bond is called "artificial interchain disulfide bond".

### Detailed description of invention

### TCR molecule

During antigen processing, antigens are degraded in cells and then carried to the cell surface by MHC molecules. T cell receptors are able to recognize peptide-MHC complexes on the surface of antigen-presenting cells. Therefore, the first aspect of the present invention provides a TCR molecule capable of binding to NLVPMVATV-HLA-A*0201 complex. Preferably, the TCR molecule is isolated or purified. The α and β chain of the TCR each has three complementarity determining regions (CDRs).

In a preferred embodiment of the present invention, the alpha chain of the TCR comprises CDRs with the following amino acid sequences:
α-CDR1: SSNFYA (SEQ ID NO.9),
α-CDR2: MTLNGDE (SEQ ID NO.11),
α-CDR3: CAFPYNNNDMRF (SEQ ID NO.13).

In a preferred embodiment of the present invention, the beta chain of the TCR comprises CDRs with the following amino acid sequences:
β-CDR 1: MNHEY (SEQ ID NO.17),
β-CDR 2: SMNVEV (SEQ ID NO.19),
β-CDR 3: CASSLEGYTEAFF (SEQ ID NO.21).

A chimeric TCR may be prepared by embedding the above CDR region amino acid sequences of the present invention into any suitable frame structure. If the framework structure is compatible with the CDR region of the TCR of the present invention, those skilled in the art can design or synthesize TCR molecules with corresponding functions according to the CDR regions of the present invention. Therefore, the TCR molecule of the present invention refers to a TCR molecule containing the above α and/or β chain CDR region sequence and any suitable framework structure.

The TCR α chain variable domain of the present invention is an amino acid sequence having at least 90%, preferably 95%, more preferably 98% sequence identity with SEQ ID No.7; and/or the TCR β chain variable domain of the present invention is an amino acid sequence having at least 90%, preferably 95%, more preferably 98% sequence identity with SEQ ID No.15.

In a preferred embodiment of the present invention, the TCR molecule of the present invention is a heterodimer composed of α and β chains. Specifically, in one aspect, the α chain of the heterodimeric TCR molecule comprises a variable domain and a constant domain, and the amino acid sequence of the α chain variable domain comprises CDR1 (SEQ ID No.9), CDR2 (SEQ ID No.11) and CDR3 (SEQ ID No.13) of the above α chain. Preferably, the TCR molecule comprises the α chain variable domain with an amino acid sequence of SEQ ID No.7. More preferably, the amino acid sequence of the α chain variable domain of the TCR molecule is SEQ ID No.7. In another aspect, the β chain of the heterodimeric TCR molecule comprises a variable domain and a constant domain, and the amino acid sequence of the β chain variable domain comprises CDR1 (SEQ ID No.17), CDR2 (SEQ ID No.19) and CDR3 (SEQ ID No.21) of the above β chain. Preferably, the TCR molecule comprises the β-chain variable domain with an amino acid sequence of SEQ ID No.15. More preferably, the amino acid sequence of the β chain variable domain of the TCR molecule is SEQ ID No.15.

In a preferred embodiment of the invention, the TCR molecule of the invention is a single chain TCR molecule consisting of part or all of the α chain and/or part or all of the β chain. For the description of single chain TCR molecules, Chung et al (1994) Proc. Natl. Acad. Sci. USA 91, 12654-12658 may be referred to. According to its description, those skilled in the art can easily construct a single chain TCR molecule containing the CDRs region of the invention. Specifically, the single chain TCR molecule comprises Vα, Vβ and Cβ, preferably connected in the order from the N-terminus to the C-terminus.

The α chain variable domain amino acid sequence of the single chain TCR molecule comprises CDR1 (SEQ ID No.9), CDR2 (SEQ ID No.11) and CDR3 (SEQ ID No.13) of the above α chain. Preferably, the single chain TCR molecule comprises the α chain variable domain amino acid sequence of SEQ ID No.7. More preferably, the amino acid sequence of the α chain variable domain of the single chain TCR molecule is SEQ ID No.7. The β chain variable domain amino acid sequence of the single chain TCR molecule comprises CDR1 (SEQ ID No.17), CDR2 (SEQ ID No.19) and CDR3 (SEQ ID No.21) of the above β chain. Preferably, the single chain TCR molecule comprises the β chain variable domain amino acid sequence of SEQ ID No.15. More preferably, the amino acid sequence of the β chain variable domain of the single chain TCR molecule is SEQ ID No.15.

In a preferred embodiment of the invention, the constant domain of the TCR molecule of the invention is the constant domain of human or mouse. Those skilled in the art know or can obtain the human constant domain amino acid sequence by consulting relevant books or the public database of IMGT (international ImMunoGeneTics information system). For example, the constant domain sequence of the α chain of the TCR molecule of the present invention can be "TRAC*01", and the constant domain sequence of TCR molecule beta chain can be "TRBC1*01" or "TRBC2*01". Preferably, the amino acid sequence of the α chain of the TCR molecule of the invention is SEQ ID No.3, and/or the amino acid sequence of the β chain is SEQ ID No.5.

The TCR of the present invention can contain an artificial disulfide bond introduced between residues of its α and β chain constant domains. It should be noted that the TCR of the present invention all can comprise TRAC constant domain sequence and TRBC1 or TRBC2 constant domain sequence with or without the introduced artificial disulfide bond as described above between the constant domains. The TRAC constant domain sequence and the TRBC1 or TRBC2 constant domain sequence of the TCR can be linked by natural disulfide bonds present in the TCR.

The TCR constant region of the present invention can be modified to prevent mismatch with endogenous TCR chains. Therefore, in the TCR of the present invention, an artificial disulfide bond is introduced between the residues of its α and β chain constant domains.

In addition, the TCR of the present invention can also be a heterozygous TCR containing sequences derived from more than one species. For example, studies have shown that murine TCR is more efficiently expressed in human T cells than human TCR. Therefore, the TCR of the present invention may comprise a human variable domain and a murine constant domain. The disadvantage of this method is that it may trigger an immune response. Therefore, there should be a regulatory scheme for immunosuppression when it is used in adoptive T cell therapy to allow the implantation of T cells expressing murine.

It should be understood that the amino acid name herein is expressed by the international single English letter or three English letters. The corresponding relationship between the single English letter and the three English letters of the amino acid name is as follows: Ala (A), Arg (R), Asn (N), Asp (D), Cys (C), Gln (Q), Glu (E), Gly (G), His (H), Ile (I), Leu (L), Lys (K), Met (M), Phe (F), Pro (P), Ser (S), Thr (T), Trp (W), Tyr (Y), Val (V).

### Nucleic acid molecule

The second aspect of the present invention provides a nucleic acid molecule encoding the TCR molecule or part thereof according to the first aspect of the present invention, said part may be one or more CDRs, variable domains of α and/or β chains, and α and/or β chains.

The nucleotide sequences encoding the CDR regions of the alpha chain of the TCR molecule of the first aspect of the present invention are as follows:
α CDR1- TCCAGCAATTTCTACGCC (SEQ ID NO.10)
α CDR2- ATGACCCTCAACGGCGATGAA (SEQ ID NO.12)
α CDR3- TGTGCTTTTCCTTATAACAACAACGATATGAGGTTC (SEQ ID NO.14)

The nucleotide sequences encoding the CDR regions of the beta chain of the TCR molecule of the first aspect of the present invention are as follows:
β CDR1- ATGAACCATGAATAC (SEQ ID NO.18)
β CDR2- TCTATGAATGTGGAGGTG (SEQ ID NO.20)
β CDR3- TGCGCTTCCTCCCTCGAGGGGTACACCGAGGCATTTTTT (SEQ ID NO.22)

Therefore, the nucleotide sequence of the nucleic acid molecule of the invention encoding the TCR α chain of the invention includes SEQ ID No. 10, SEQ ID No.12 and SEQ ID No.14, and/or the nucleotide sequence of the nucleic acid molecule of the invention encoding the TCR β chain of the invention includes SEQ ID No.18, SEQ ID No.20 and SEQ ID No.22.

The nucleotide sequence of the nucleic acid molecule of the invention can be single stranded or double stranded, the nucleic acid molecule can be RNA or DNA, and can contain introns or not. Preferably, the nucleotide sequence of the nucleic acid molecule of the invention does not contain introns but can encode the polypeptides of the invention. For example, the nucleotide sequence of the nucleic acid molecule of the invention encoding the TCR α chain variable domain of the invention includes SEQ ID No.8 and/or the nucleotide sequence of the nucleic acid molecule of the invention encoding the TCR β chain variable domain of the invention includes SEQ ID No.16. More preferably, the nucleotide sequence of the nucleic acid molecule of the present invention comprises SEQ ID No.4 and/or SEQ ID No.6. Alternatively, the nucleotide sequence of the nucleic acid molecule of the present invention is SEQ ID No.2.

It should be understood that due to the degeneracy of the genetic code, different nucleotide sequences can encode the same polypeptide. Therefore, the nucleic acid sequence encoding the TCR of the present invention may be the same as or degenerate variant of the nucleic acid sequence shown in the accompanying drawings of the present invention. To illustrate with one of the examples in the present invention, "degenerate variant" refers to a nucleic acid sequence coding for a protein sequence with SEQ ID No. 1, but different from the sequence of SEQ ID No. 2.

The nucleotide sequence can be codon optimized. Different cells have different use of specific codons. According to the type of cell, the codons in the sequence can be changed to increase the expression. The codon selection table of mammalian cells and a variety of other organisms is well known to those skilled in the art.

The full-length sequence or fragments of the nucleic acid molecule of the present invention can generally be obtained by but not limited to PCR amplification, recombination or artificial synthesis. At present, the DNA sequence encoding the TCR (or its fragment, or its derivative) of the present invention can be obtained completely through chemical synthesis. Then this DNA sequence can be introduced into various existing DNA molecules (or such as vectors) and cells known in the art. DNA can be coding chain or noncoding chain.

### Vector

The invention also relates to vectors containing nucleic acid molecules of the invention, including expression vectors, that is, constructs that can be expressed in vivo or in vitro. Commonly used vectors include bacterial plasmids, phages, and animal and plant viruses.

Virus delivery systems include but are not limited to adenoviral vectors, adeno-associated virus (AAV) vectors, herpesvirus vectors, retroviral vectors, lentiviral vectors, and baculovirus vectors.

Preferably, the vector can transfer the nucleotide of the present invention into a cell, such as a T cell, so that the cell expresses an antigen-specific TCR. Ideally, the vector should be able to be expressed at high and sustained levels in T cells.

### Cell

The invention also relates to host cells generated by genetic engineering with the vector or coding sequence of the invention. The host cell contains the vector of the invention or the nucleic acid molecule of the invention is integrated in the chromosome. Host cells are selected from prokaryotic cells and eukaryotic cells, such as *Escherichia coli,* yeast cells, CHO cells, 293T cells, etc.

In addition, the invention also includes isolated cells expressing the TCR of the invention, especially T cells. The T cells may be derived from T cells isolated from the subject or may be a mixed population of cells isolated from the subject, such as part of the peripheral blood lymphocyte (PBL) population. For example, the cells can be isolated from peripheral blood mononuclear cells (PBMCs), which can be CD4⁺ helper T cells or CD8⁺ cytotoxic T cells. The cells can be in the mixed population of CD4⁺ helper T cells/CD8⁺ cytotoxic T cells.

Alternatively, the cells of the present invention may also be or be derived from stem cells, such as hematopoietic stem cells (HSCs). Gene transfer to HSCs does not result in the expression of TCR on the cell surface, because CD3 molecules are not expressed on the surface of stem cells. However, when stem cells differentiate into lymphoid precursors that migrate to the thymus, the expression of CD3 molecules will initiate the expression of the introduced TCR molecules on the surface of thymocytes.

There are many methods suitable for T cell transfection with DNA or RNA encoding the TCR of the present invention (e.g., Robbins et al, (2008) J. Immunol.180:6116-6131). T cells expressing the TCR of the present invention can be used for adoptive immunotherapy. Those skilled in the art can know many suitable methods for adoptive treatment (e.g., Rosenberg et al, (2008) Nat Rev Cancer8 (4): 299-308).

### Cytomegalovirus (CMV) related disease

The invention also relates to a method for treating and/or preventing CMV related diseases in a subject, which comprises a step of adoptive transfer of CMV specific T cells to the subject. The CMV specific T cell can recognize the major CMV matrix phosphoprotein pp65. The CMV specific T cell can recognize the epitope NLVPMVATV.

CMV is a ubiquitous human herpesvirus that infects about 50% of normal individuals. In most cases, the immune response can control acute infection by recognizing CMV derived antigens. The virus then remains latent throughout the life of the host. Outgrowth is prevented by effector mechanisms of the immune system, including neutralizing antibodies against viral membrane proteins, HLA restricted CMV specific helper and cytotoxic T cells, and MHC restricted effectors.

CMV infection is important for some high-risk populations. The main scope of infection risk includes pre or post natal infants, as well as immune compromised individuals, such as organ transplant recipients, leukemia patients or people infected with human immunodeficiency virus (HIV).

There are generally three clinical forms of CMV infection, including:
(1) Neonatal CMV inclusion disease, which may be a serious disease that affects the liver, spleen and central nervous system and may cause disability from asymptomatic;
(2) Acute acquired CMV infection, which is similar to infectious mononucleosis, which shows fever, discomfort, skeletal muscle pain and other symptoms;
(3) CMV infection in immune compromised individuals (for example, people who have transplanted organs or people with HIV), which has the risk of CMV retinitis, CMV pneumonia, CMV gastroenteritis and CMV encephalitis.

The TCR of the invention can be used to treat and/or prevent the reactivation of latent CMV after allogeneic hematopoietic stem cell transplantation.

CMV disease in allo-HSCT recipients is thought to arise primarily from reactivation of latent viruses. Transmission of the virus can occur from donor bone marrow infusion or from allogeneic blood products. In immune compromised bone marrow transplant recipients, viral reactivation usually leads to progressive CMV infection, which is the main cause of infectious morbidity and mortality in this patient population. Progressive CMV infection is the result of both immunosuppression and delayed immune recovery in these patients after transplantation.

In the method of the present invention, for example, the T cells expressing CMV specific T cell receptors of the present invention are applied to carry out adoptive immunotherapy for Allo-HSCT recipients.

### Method of prevention and treatment

The term "prevention" refers to avoiding, delaying, impeding or hindering the progression of a disease. For example, the possibility of CMV infection and/or CMV reactivation can be prevented or reduced.

The "treatment" used herein refers to relieving, curing or reducing the symptoms of a disease, or reducing or preventing the progression of a disease.

Prevention and treatment may be conducted by isolating T cells from patients or volunteers with related diseases, introducing the TCR of the invention into the above T cells, and then reinfusing these genetically modified cells into the patient.

Therefore, the invention provides a method for treating CMV related diseases, which comprises inputting isolated T cells expressing the TCR of the invention, preferably the T cells are from a patient, into the patient. Generally, it comprises:
(1) Isolating T cells of the patient;
(2) Transducing the T cells *in vitro* with nucleic acid molecules of the invention or nucleic acid molecules capable of encoding TCR molecules of the invention;
(3) Infusing genetically modified T cells into the patient.

The number of cells isolated, transfected and reinfused can be determined by the physician.

**The main advantages of the present invention are:**
(1) The TCR of the invention can rapidly clear CMV virus directly through precise targeting;
(2) The TCR of the invention has high protein expression *in vivo* and no mismatch with the endogenous TCR chain;
(3) The T cells expressing the TCR of the invention have strong killing ability and specificity, and can be effectively used for the treatment of CMV infection in hematopoietic stem cell transplantation (HSCT) patients or other transplant patients.

The following specific examples further elaborate the present invention. It should be understood that these examples are only used to illustrate the invention and not to limit the scope of the invention. The experimental methods without specific conditions indicated in the following examples are usually based on conventional conditions, such as the conditions described in Molecular Cloning-A Laboratory Manual (Third Edition) (2001) CSHL press, Sambrook and Russell *et al.,* or the conditions recommended by the manufacturer. Unless otherwise stated, percentages and portions are calculated by weight. The experimental materials and reagents used in the following examples can be obtained from commercially available sources without special instructions.

### Example 1. Induction of differentiation of monocytes into dendritic cells (DC cells) in vitro

(1) PBMCs in the blood of healthy donors/patients were obtained by Lymphoprep density gradient centrifugation (or frozen PBMCs were resuscitated), after resuspension with cytotoxic T lymphocyte medium, the supernatant was removed by centrifugation at 350 g for 5 min, and appropriate amount of DC cell culture medium was added to resuspend PBMCs into 6-well plates. After culturing in the incubator for 2 h, the medium and clustered non-adherent cells were aspirated.
(2) Each well was gently added with 3 ml of DC medium containing final concentration of 1×DC Differentiation Supplement (GM-CSF and IL-4) along the side wall, and the 6-well plate was placed in a 5% CO₂ incubator at 37°C for 5 days.
(3) On the fifth day, without replacing the culture medium, 30 µl of 100×DC Maturation Supplement was added to the culture medium per well (the final concentration of DC Maturation Supplement is 1×). The 6-well plate was placed in a 5% CO₂ incubator at 37°C to induce maturation for 2 d.
(4) Mature DC cells can be harvested on the seventh day. The cells at the bottom of the well plate were vigorously blown and resuspended with a pipette to collect mature DC cells. The collected mature DC cells were transferred to a 15 ml centrifuge tube, and the supernatant was removed after centrifugation at 350 g for 5 min. The DCs were resuspended using DC medium at a density of 2 × 10⁶ cells/ml for subsequent experiments.

### Example 2. in vitro stimulation and expansion of human CMV-pp65 antigen-specific T cells

(1) CMV-pp65 target peptide of 5 µg/ml was added to the above mature DC cells. It was sealed with parafilm and put into a rotator (MACSmix) and rotated and binding in a cell incubator for 4 h. After the incubation of mDCs with CMV-pp65 target peptide (CMV-peptide), DC cells loaded with CMV-pp65 target peptide (CMV-peptide-loaded DCs) were irradiated with an irradiator at a dose of 40 Gy. After irradiation, DC cells loaded with CMV-pp65 target peptide were collected by centrifugation into a 15 ml centrifuge tube.
(2) PBMCs in the blood of healthy donors were obtained by lymphoprep density gradient centrifugation. CD8+ T cells from PBMCs were isolated by using a kit (EasySep^{™} Human CD8 Positive Selection Kit). At a DC/T ratio of 1:2.5, DC cells loaded with CMV-pp65 target peptide (CMV-peptide-loaded DCs) and CD8+ T cells were added to 12-well plates for culture. IL-21 with a final concentration of 30 ng/ml and CMV-PP65 target peptide(CMV-PP65 peptide) with a final concentration of 5 µg/ml were added to the culture system.
(3) The 12-well plates were incubated overnight at 37 °C in a 5% CO₂ cell incubator. On the first day, 10 ng/ml of IL-2, IL-7, and IL-15 were added to each well, and then placed in an cell incubator for 10 days at 37 °C and 5% CO₂. During the culture process, half of the solution was changed every 2-3 days. On the 5th and 10th day, CMV-PP65 target peptide (CMV-PP65 peptide) was added to each culture system at a final concentration of 5 µg/ml.
(4) On the 11th day, referring to the above steps, the irradiated DC cells loaded with CMV-pp65 target peptide (irradiated CMV-peptide-loaded DCs) were prepared for the second round of in vitro stimulation with the cells in each culture system, and the stimulation continued for 7 days.
(5) On the 18th day, 500 µl cells were taken from each well in the well plate, washed twice with buffer solution(FACS buffer), CMV-PP65-HLA:A:0201 Dextramer-FITC and CD8a-PE was added, mixed well, and incubated in the dark at 4 °C for 20 min. After incubation, they were washed three times with FACS buffer, and then the cells were resuspended with 400 µl buffer solution (FACS buffer) containing 1 × DAPI working solution, and CD8+/Dextramer+ (%) ratio was detected.

### Example 3. single cell sequencing to obtain HLA:A:0201 restricted CMV-pp65 antigen-specific TCR

(1) CD8+/Dextramer+ T cells were sorted by flow cytometry, and the TCR α and TCR β chain sequences of CD8+/Dextramer+ T cells were obtained by 10X Genomics single-cell sequencing technology.
(2) After the TCR sequence was obtained by single-cell sequencing, the TCR α/β chain sequence with the highest frequency was optimized, and its constant region was modified to avoid the mismatch between the exogenous TCR and the endogenous TCR of T cells; the expression sequence was codon optimized to improve the protein expression; using P2A and Furin protease cleavage sites(Furin-cleavage), TCR α and TCR β could be simultaneously expressed in an expression vector without producing redundant proline tails.
(3) The lentiviral expression vector was constructed, and the above TCRα and TCRβ sequences were inserted into the same lentiviral expression vector. The virus was packaged by 293T cells to produce CMV-pp65 antigen-specific TCR specific viral particles.

### Example 4. Infection of CMV-pp65-TCR lentivirus to activate T cells to produce CMV-pp65-TCR-T

(1) CD8+ T cells in PBMCs were isolated using a kit (EasySep^{™} Human CD8 Positive Selection Kit). CD8+ T cells were activated using CD3/28 immunomagnetic beads (Dynabeads) at a ratio of 1:3 CD8+ T cell:CD3/28 immunomagnetic beads (Dynabeads) in vitro, and CD8+ T cells were stimulated and activated at 37 °C for 2 d.
(2) CMV-pp65-TCR lentiviral particles were used to infect activated CD8+ T cells. 8 µg/ml polybrene was added, 500Xg, 90 min, 30 °C for centrifugation infection. After centrifugation, the cells were placed in a cell incubator and cultured at 37 °C with 5% CO₂ for 3 days, followed by a second round of infection with CMV-pp65-TCR lentivirus.
(3) Three days after the second round of infection with CMV-pp65-TCR lentivirus, CMV-pp65-TCR-T cells were aspirated, washed twice with buffer solution (FACS buffer), CMV-PP65-HLA*A:0201 Dextramer-FITC and CD8a-PE antibody were added for staining, the ratio of CD8+/Dextramer+ (%) was detected by FACS, and then CD8a+/Dextramer+ T cells were sorted by flow cytometry for subsequent in vitro killing experiments.

### Example 5. HLA:A*0201 restricted CMV-pp65-TCR-T activation experiment in vitro

### A. Detection of expression levels of CD137, IFNy and TNF-α in TCR-T cells by FACS

(1) K562-A:0201-GFP, K562-A:0201-GFP-mCherry-vector and K562-A:0201-GFP-pp65-mCherry cells were prepared by lentivirus infection of K562 cells, respectively;
(2) CMV-pp65-TCR-T/Reported-TCR-T cells prepared in Example 4 were respectively inoculated with TCR-T cells and K562 cells according to the effector-target ratio of 1:1 to round bottom 96 well plates for culture.
(3) After 24 h of coculture, the cells were stained extracellularly, fixed and broken. After intracellular staining, the expression levels of CD137, TNF α and IFN γ of CD8+T cells in the coculture system were detected by FACS to evaluate whether K562-A:0201-GFP-pp65-mCherry target cells could specifically activate CMV-pp65-TCR-T cells in vitro.

### B. Detection of the release level of IFNy from TCR-T cells by Elispot

(1) The coating antibody (MabtechIFNγ, 1-D1K: 1 mg/ml) was diluted to 15 µg/ml (1.5 µg/100 µl) using sterile 1 × DPBS without calcium and magnesium ions at pH 7.4. 100 µl diluted 1-D1K antibody solution was added to each well and incubated overnight at 4-8 °C.
(2) The antibody was removed from the plate, after washing with sterile DPBS, the cells were plated at an effector-target ratio of 1:1, 100 µl of 1640 complete medium, 3 × 10⁴ CMV-pp65-TCR-T cells or K562 cells were added to each well, the edge of the plate was tapped to make the cells spread evenly, and it was put into the incubator, incubated at 37°C and 5% CO₂ for 20 h.
(3) The cells were removed after 20 h, washed with DPBS for 4-6 times to completely remove the cells, and then 100 µl of biotin labeled detection antibody 7-B6-1-biotin was added to each well and incubated at 37°C for 2 h. After incubation, the plate was washed with DPBS for five times, the liquid was discarded for the last time, and gently dried on sterile paper. Subsequently, 200 µl Streptavidin-HRP was added to each well and incubated at 37°C for 1 h.
(4) After the incubation, the plate was washed with DPBS for 5 times, and 100 µl of substrate TMB was added to each well until clear spots appeared. Finally, deionized water was added to stop the color development reaction. Statistical analysis was performed after scanning the plate with Elispots-reader.

### Example 6. HLA:A*0201 restricted CMV-pp65-TCR-T specifically kills pp65-HLA*A0201-K562 cells in vitro

(1) K562-A:0201-GFP control cells and K562-A:0201-GFP-pp65-mCherry target cells were prepared by lentivirus infection of K562 cells, respectively;
(2) CMV-pp65-TCR-T/Reported-TCR-T cells prepared in Example 4 were takenand the T cells and K562 cells were inoculated at the effector-target ratios of 1:1 and 5:1 respectively to round bottom 96 well plates for culture.
(3) After 24 h of coculture, the proportion of K562-A:0201-GFP or K562-A:0201-GFP-pp65-mCherry in the coculture system was detected by FACS to evaluate whether CMV-pp65-TCR-T and Reported-TCR-T could specifically kill K562-A:0201-GFP-pp65-mCherry cells, and the killing efficiency of the two TCR-Ts was compared.

### Example 7. HLA:A*0201 restricted CMV-pp65-TCR-T specifically kills pp65-HLA*A0201-K562 cells in vivo

(1) K562-A:0201-GFP-pp65-luciferase target cells were prepared by lentivirus infection of K562 cells; and CMV-pp65-TCR-T effector cells were prepared by lentiviral infection of CD8⁺T cells.
(2) Subcutaneous tumor implantation model in mice: 1 × 10⁶ target cells were subcutaneously injected into the right flank of NOG mice on Day 0, 5 × 10⁶ primary CD8⁺ T cells (CD8⁺T day 3 group) and CMV-pp65-TCR-T cells (pp65-TCR-T day 3 group) were injected through the lateral tail vein on Day 3, and 5 × 10⁶ CMV-pp65-TCR-T cells (pp65-TCR-T day 37 group) were injected through the lateral tail vein on Day 7; at d0 (+4h), d7, d14, d21, d28, d35 after tumor cell injection, mice were subjected to bioluminescence imaging to detect the expression of luciferase in tumor cells, and the photon number intensity of luciferase was quantified to evaluate whether CMV-pp65-TCR-T could inhibit the growth of K562-A:0201-GFP-pp65-luciferase target cells.
(3) Mouse tail vein metastasis model: 1×10⁶ target cells were injected into the lateral tail vein of NOG mice on day 0, and 1×10⁷ primary CD8⁺ T cells and CMV-pp65-TCR-T cells were injected through the lateral tail vein on day 3. At d0 (+4h), d28, d35, d42, d49, d56 after tumor cell injection, mice were subjected to bioluminescence imaging to detect the expression of luciferase in tumor cells, and the photon number intensity of luciferase was quantified to evaluate the inhibitory effect of CMV-pp65-TCR-T on target cell K562-A:0201-GFP-pp65-luciferase.

### Experimental results:

### Screening process of CMV-pp65-TCR-T:

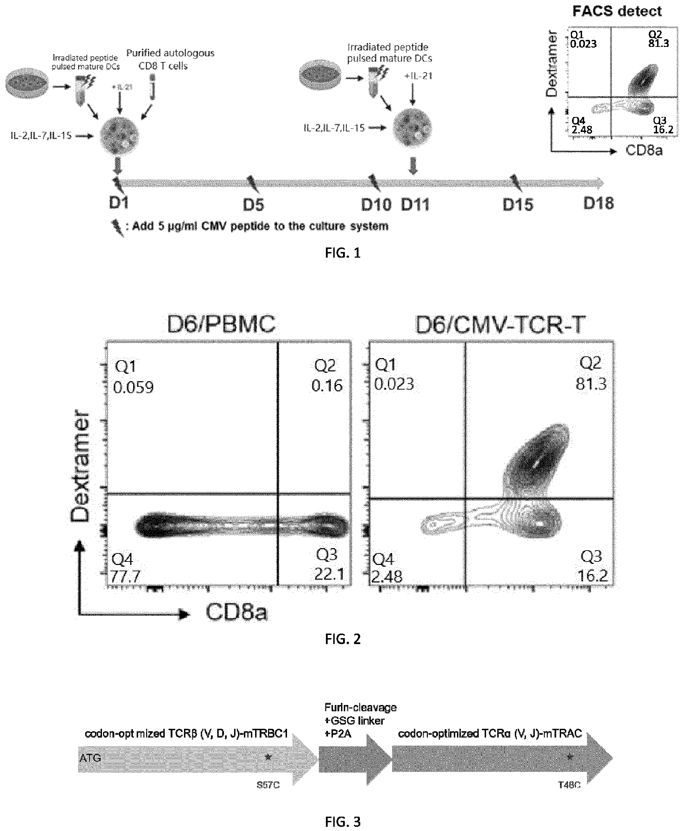

FIG. 1 shows the specific stimulation and screening process of the CMV-pp65-TCR-T of the invention. The whole test was conducted by CD8⁺ T cells from healthy HLA-A:0201 donors after two rounds of stimulation with autologous mDCs loaded with pp65 polypeptides, and then CMV-pp65-HLA*A:0201 Dextramer was used to analyze the specificity of CMV-pp65 antigen-specific TCR-T cells.

The flow cytometry results (FIG. 2) shows that after two rounds of mDCs-pp65 polypeptide stimulation, the CD8a⁺/Dextramer⁺ T ratio of healthy donor D6 increased from 0.16% to 81.3%, indicating that CMV-pp65-specific TCR-T cells were specifically expanded in vitro.

### Obtaining CMV-pp65-TCR sequences by single-cell sequencing:

After two rounds of stimulation with autologous mDCs loaded with pp65 polypeptides, CMV-PP65-HLA*A:0201 Dextramer and CD8a antibodies were used to perform flow sorting of CMV-pp65 antigen-specific TCR-T cells. The sorted CMV-pp65-TCR-T cells were subjected to single-cell sequencing by 10×Genomics method to obtain the α and β chain sequences of CMV-pp65-TCR-T cells.

After single-cell sequencing, the top 10 frequencies obtained by sequencing were sorted. Single-cell sequencing results showed that 99.64% of the T cell clones in the sorted CD8a⁺/Dextramer⁺ T cells were composed of a TCR-T, which proved that the specificity of this experiment (Antigen specific T cell priming) was good. This T cell clone contains two α chains, one of which is redundant α chain, which has been excluded by *in vitro* CMV-PP65-HLA*A:0201 Dextramer binding experiment.

The final obtained TCR sequence is as follows:
CMV-pp65-TCR:
MGPQLLGYVVLCLLGAGPLEAQVTQNPRYLITVTGKKLTVTCSQNMNH EYMSWYRQDPGLGLRQIYYSMNVEVTDKGDVPEGYKVSRKEKRNFPLILES PSPNQTSLYFCASSLEGYTEAFFGQGTRLTVVEDLRNVTPPKVSLFEPSKAEIA NKQKATLVCLARGFFPDHVELSWWVNGKEVHSGVCTDPQAYKESNYSYCL SSRLRVSATFWHNPRNHFRCQVQFHGLSEEDKWPEGSPKPVTQNISAEAWG RADCGITSASYQQGVLSATILYEILLGKATLYAVLVSTLVVMAMVKRKNSRA KRGSGATNFSLLKQAGDVEENPGPMEKNPLAAPLLILWFHLDCVSSILNVEQ SPQSLHVQEGDSTNFTCSFSSSNFYALHWYRWETAKSPEALFVMTLNGDEK KKGRISATLNTKEGYSYLYIKGSQPEDSATYLCAFPYNNNDMRFGAGTRLTV EPDIQNPEPAVYQLKDPRSQDSTLCLFTDFDSQINVPKTMESGTFITDKCVLD MKAMDSKSNGATAWSNQTSFTCQDIFKETNATYPSSDVPCDATLTEKSFETD MNLNFQNLSVMGLRILLLKVAGFNLLMTLRLWSS (SEQ ID NO.1), the C in the sequence is a cysteine residue introduced by artificial mutation;
The TCR coding sequence after codon optimization is as follows:

ATGGGGCCCCAGCTTTTGGGTTATGTAGTTCTTTGTCTCCTGGGCGCT
GGACCGCTGGAAGCACAAGTCACTCAAAACCCTAGGTACCTGATCACCGT
GACCGGAAAGAAGCTGACTGTGACCTGCTCTCAGAACATGAACCATGAA
TACATGTCATGGTACAGGCAGGATCCTGGGCTCGGACTTAGACAGATTTA
CTACTCTATGAATGTGGAGGTGACAGATAAAGGGGATGTGCCAGAAGGC
TATAAGGTCTCCAGGAAGGAGAAGAGAAACTTTCCCTTGATCTTGGAGTC
TCCGTCCCCAAACCAGACCTCTCTGTACTTCTGCGCTTCCTCCCTCGAGGG
GTACACCGAGGCATTTTTTGGACAGGGGACTAGACTGACCGTGGTGGAGG
ATCTGAGAAATGTGACTCCACCCAAGGTCTCCTTGTTTGAGCCATCAAAA
GCAGAGATTGCAAACAAACAAAAGGCTACCCTCGTGTGCTTGGCCAGGG
GCTTCTTCCCTGACCACGTGGAGCTGAGCTGGTGGGTGAATGGCAAGGAG
GTCCACAGTGGGGTCTGCACGGACCCTCAGGCCTACAAGGAGAGCAATT
ATAGCTACTGCCTGAGCAGCCGCCTGAGGGTCTCTGCTACCTTCTGGCAC
AATCCTCGCAACCACTTCCGCTGCCAAGTGCAGTTCCATGGGCTTTCAGA
GGAGGACAAGTGGCCAGAGGGCTCACCCAAACCTGTCACACAGAACATC
AGTGCAGAGGCCTGGGGCCGAGCAGACTGTGGGATTACCTCAGCATCCTA
TCAACAAGGGGTCTTGTCTGCCACCATCCTCTATGAGATCCTGCTAGGGA
AAGCCACCCTGTATGCTGTGCTTGTCAGTACACTGGTGGTGATGGCTATG
GTCAAAAGAAAGAATTCAAGAGCCAAAAGAGGTTCTGGCGCCACCAACT
TCAGCCTGCTGAAGCAGGCCGGCGACGTGGAGGAGAACCCCGGCCCCAT
GGAAAAGAACCCTCTGGCTGCCCCCCTGTTGATACTGTGGTTCCACCTGG
ACTGCGTGAGCAGCATTCTGAACGTCGAACAGTCCCCTCAGTCTCTGCAC
GTCCAAGAGGGAGATTCCACCAATTTTACTTGTAGTTTTAGCTCCAGCAA
TTTCTACGCCCTGCACTGGTACCGGTGGGAGACCGCCAAGTCTCCCGAAG
CCCTCTTTGTGATGACCCTCAACGGCGATGAAAAAAAAAAAGGGAGAAT
TAGCGCCACCCTGAACACTAAAGAGGGCTATTCTTACTTGTATATAAAAG
GGTCTCAGCCTGAGGACAGCGCTACATACTTGTGTGCTTTTCCTTATAACA
ACAACGATATGAGGTTCGGGGCCGGAACAAGGTTGACCGTTGAGCCTGA
CATCCAGAACCCAGAACCTGCTGTGTACCAGTTAAAAGATCCTCGGTCTC
AGGACAGCACCCTCTGCCTGTTCACCGACTTTGACTCCCAAATCAATGTG
CCGAAAACCATGGAATCTGGAACGTTCATCACTGACAAATGCGTGCTGGA
CATGAAAGCTATGGATTCCAAGAGCAATGGGGCCATTGCCTGGAGCAAC
CAGACAAGCTTCACCTGCCAAGATATCTTCAAAGAGACCAACGCCACCTA
CCCCAGTTCAGACGTTCCCTGTGATGCCACGTTGACCGAGAAAAGCTTTG

AAACAGATATGAACCTAAACTTTCAAAACCTGTCAGTTATGGGACTCCGA
ATCCTCCTGCTGAAAGTAGCGGGATTTAACCTGCTCATGACGCTGAGGCT
GTGGTCCAGT (SEQ ID NO.2)

Wherein,
The α chain and its coding sequence are as follows:

MEKNPLAAPLLILWFHLDCVSSILNVEQSPQSLHVQEGDSTNFTCSFSSS
NFYALHWYRWETAKSPEALFVMTLNGDEKKKGRISATLNTKEGYSYLYIKG
SQPEDSATYLCAFPYNNNDMRFGAGTRLTVEPDIQNPEPAVYQLKDPRSQDS
TLCLFTDFDSQINVPKTMESGTFITDKCVLDMKAMDSKSNGAIAWSNQTSFT
CQDIFKETNATYPSSDVPCDATLTEKSFETDMNLNFQNLSVMGLRILLLKVA
GFNLLMTLRLWSS (SEQ ID NO.3)

ATGGAAAAGAACCCTCTGGCTGCCCCCCTGTTGATACTGTGGTTCCA
CCTGGACTGCGTGAGCAGCATTCTGAACGTCGAACAGTCCCCTCAGTCTC
TGCACGTCCAAGAGGGAGATTCCACCAATTTTACTTGTAGTTTTAGCTCC
AGCAATTTCTACGCCCTGCACTGGTACCGGTGGGAGACCGCCAAGTCTCC
CGAAGCCCTCTTTGTGATGACCCTCAACGGCGATGAAAAAAAAAAAGGG
AGAATTAGCGCCACCCTGAACACTAAAGAGGGCTATTCTTACTTGTATAT
AAAAGGGTCTCAGCCTGAGGACAGCGCTACATACTTGTGTGCTTTTCCTT
ATAACAACAACGATATGAGGTTCGGGGCCGGAACAAGGTTGACCGTTGA
GCCTGACATCCAGAACCCAGAACCTGCTGTGTACCAGTTAAAAGATCCTC
GGTCTCAGGACAGCACCCTCTGCCTGTTCACCGACTTTGACTCCCAAATC
AATGTGCCGAAAACCATGGAATCTGGAACGTTCATCACTGACAAATGCGT
GCTGGACATGAAAGCTATGGATTCCAAGAGCAATGGGGCCATTGCCTGG
AGCAACCAGACAAGCTTCACCTGCCAAGATATCTTCAAAGAGACCAACG
CCACCTACCCCAGTTCAGACGTTCCCTGTGATGCCACGTTGACCGAGAAA
AGCTTTGAAACAGATATGAACCTAAACTTTCAAAACCTGTCAGTTATGGG
ACTCCGAATCCTCCTGCTGAAAGTAGCGGGATTTAACCTGCTCATGACGC
TGAGGCTGTGGTCCAGT (SEQ ID NO.4);

The β chain and its coding sequence are as follows:

MGPQLLGYVVLCLLGAGPLEAQVTQNPRYLITVTGKKLTVTCSQNMNH
EYMSWYRQDPGLGLRQIYYSMNVEVTDKGDVPEGYKVSRKEKRNFPLILES
PSPNQTSLYFCASSLEGYTEAFFGQGTRLTVVEDLRNVTPPKVSLFEPSKAEIA
NKQKATLVCLARGFFPDHVELSWWVNGKEVHSGVCTDPQAYKESNYSYCL
SSRLRVSATFWHNPRNHFRCQVQFHGLSEEDKWPEGSPKPVTQNISAEAWG
RADCGITSASYQQGVLSATILYEILLGKATLYAVLVSTLVVMAMVKRKNS
(SEQ ID NO.5)

ATGGGGCCCCAGCTTTTGGGTTATGTAGTTCTTTGTCTCCTGGGCGCT
GGACCGCTGGAAGCACAAGTCACTCAAAACCCTAGGTACCTGATCACCGT
GACCGGAAAGAAGCTGACTGTGACCTGCTCTCAGAACATGAACCATGAA
TACATGTCATGGTACAGGCAGGATCCTGGGCTCGGACTTAGACAGATTTA
CTACTCTATGAATGTGGAGGTGACAGATAAAGGGGATGTGCCAGAAGGC
TATAAGGTCTCCAGGAAGGAGAAGAGAAACTTTCCCTTGATCTTGGAGTC
TCCGTCCCCAAACCAGACCTCTCTGTACTTCTGCGCTTCCTCCCTCGAGGG
GTACACCGAGGCATTTTTTGGACAGGGGACTAGACTGACCGTGGTGGAGG
ATCTGAGAAATGTGACTCCACCCAAGGTCTCCTTGTTTGAGCCATCAAAA
GCAGAGATTGCAAACAAACAAAAGGCTACCCTCGTGTGCTTGGCCAGGG
GCTTCTTCCCTGACCACGTGGAGCTGAGCTGGTGGGTGAATGGCAAGGAG

GTCCACAGTGGGGTCTGCACGGACCCTCAGGCCTACAAGGAGAGCAATT
ATAGCTACTGCCTGAGCAGCCGCCTGAGGGTCTCTGCTACCTTCTGGCAC
AATCCTCGCAACCACTTCCGCTGCCAAGTGCAGTTCCATGGGCTTTCAGA
GGAGGACAAGTGGCCAGAGGGCTCACCCAAACCTGTCACACAGAACATC
AGTGCAGAGGCCTGGGGCCGAGCAGACTGTGGGATTACCTCAGCATCCTA
TCAACAAGGGGTCTTGTCTGCCACCATCCTCTATGAGATCCTGCTAGGGA
AAGCCACCCTGTATGCTGTGCTTGTCAGTACACTGGTGGTGATGGCTATG
GTCAAAAGAAAGAATTCA (SEQ ID NO.6)

The α chain variable region and its coding sequence are as follows:

MEKNPLAAPLLILWFHLDCVSSILNVEQSPQSLHVQEGDSTNFTCSFSSS
NFYALHWYRWETAKSPEALFVMTLNGDEKKKGRISATLNTKEGYSYLYIKG
SQPEDSATYLCAFPYNNNDMRFGAGTRLTVEP (SEQ ID NO.7)

ATGGAAAAGAACCCTCTGGCTGCCCCCCTGTTGATACTGTGGTTCCA
CCTGGACTGCGTGAGCAGCATTCTGAACGTCGAACAGTCCCCTCAGTCTC
TGCACGTCCAAGAGGGAGATTCCACCAATTTTACTTGTAGTTTTAGCTCC
AGCAATTTCTACGCCCTGCACTGGTACCGGTGGGAGACCGCCAAGTCTCC
CGAAGCCCTCTTTGTGATGACCCTCAACGGCGATGAAAAAAAAAAAGGG
AGAATTAGCGCCACCCTGAACACTAAAGAGGGCTATTCTTACTTGTATAT
AAAAGGGTCTCAGCCTGAGGACAGCGCTACATACTTGTGTGCTTTTCCTT
ATAACAACAACGATATGAGGTTCGGGGCCGGAACAAGGTTGACCGTTGA
GCCT (SEQ ID NO.8)

The 3 CDR sequences contained are as follows:
α-CDR1: SSNFYA (SEQ ID NO.9), its coding sequence is: TCCAGCAATTTCTACGCC (SEQ ID NO.10)
α-CDR2: MTLNGDE (SEQ ID NO.11), its coding sequence is: ATGACCCTCAACGGCGATGAA (SEQ ID NO.12)
α-CDR3: CAFPYNNNDMRF (SEQ ID NO.13), its coding sequence is: TGTGCTTTTCCTTATAACAACAACGATATGAGGTTC (SEQ ID NO.14).

The β chain variable region and its coding sequence are as follows:

MGPQLLGYVVLCLLGAGPLEAQVTQNPRYLITVTGKKLTVTCSQNMNH
EYMSWYRQDPGLGLRQIYYSMNVEVTDKGDVPEGYKVSRKEKRNFPLILES
PSPNQTSLYFCASSLEGYTEAFFGQGTRLTVV (SEQ ID NO.15)

ATGGGGCCCCAGCTTTTGGGTTATGTAGTTCTTTGTCTCCTGGGCGCT
GGACCGCTGGAAGCACAAGTCACTCAAAACCCTAGGTACCTGATCACCGT
GACCGGAAAGAAGCTGACTGTGACCTGCTCTCAGAACATGAACCATGAA
TACATGTCATGGTACAGGCAGGATCCTGGGCTCGGACTTAGACAGATTTA
CTACTCTATGAATGTGGAGGTGACAGATAAAGGGGATGTGCCAGAAGGC
TATAAGGTCTCCAGGAAGGAGAAGAGAAACTTTCCCTTGATCTTGGAGTC
TCCGTCCCCAAACCAGACCTCTCTGTACTTCTGCGCTTCCTCCCTCGAGGG
GTACACCGAGGCATTTTTTGGACAGGGGACTAGACTGACCGTGGTG (SEQ
ID NO.16)

The 3 CDR sequences contained are as follows:
β-CDR 1: MNHEY (SEQ ID NO.17), its coding sequence is: ATGAACCATGAATAC (SEQ ID NO.18);
β-CDR 2: SMNVEV (SEQ ID NO.19), its coding sequence is: TCTATGAATGTGGAGGTG (SEQ ID NO.20);
β-CDR 3: CASSLEGYTEAFF (SEQ ID NO.21), its coding sequence is: TGCGCTTCCTCCCTCGAGGGGTACACCGAGGCATTTTTT (SEQ ID NO.22).

### Construction of CMV-pp65-TCR-T vector and sequence optimization:

After the sequence of pp65 TCR (SEQ ID No.1) was obtained by sequencing, the sequence was optimized, including:
(1) Modifying the constant region to prevent mismatch with endogenous TCR chain;
(2) Optimizing the expression sequence with human codon to improve the expression of TCR protein;
(3) Using P2A and Furin-cleavage to express alpha chain and beta chain simultaneously on one plasmid without producing the terminal proline residue caused by using P2A alone.

Subsequently, the optimized pp65-TCR sequence (FIG. 3) was cloned into the lentiviral expression vector.

### In vitro CMV-pp65-TCR-T binding experiment:

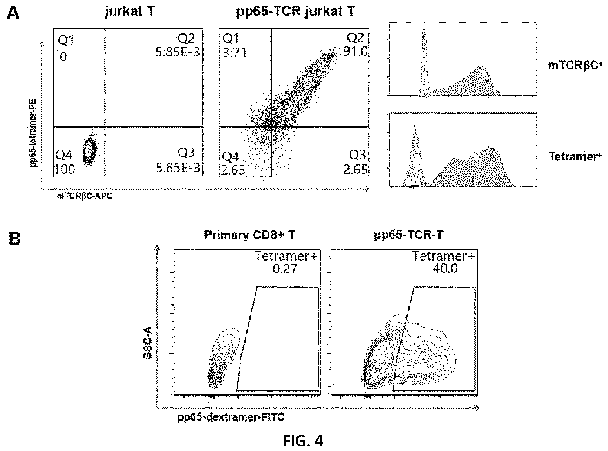

293T cells were used for packaging of TCR lentivirus. Jurkat T or PBMCs of healthy HLA-A:0201 donors were infected with CMV-pp65-TCR lentivirus for two rounds, and then CMV-pp65-HLA*A:0201 Dextramer staining was performed. Flow cytometry results showed that Tetramer⁺/mTCRβC⁺jurkatT (FIG. 4, A) and Dextramer⁺/primary CD8⁺ T (FIG. 4, B) cells clustered significantly. The results showed that CMV-pp65-TCR-T screened and identified in this project was normally expressed in jurkatT and primary CD8⁺ T cells of healthy donors and could bind to CMV-PP65-HLA*A:0201 Tetramer/Dextramer.

### In vitro CMV-pp65-TCR-T activation experiment:

The control cell K562-A:0201-GFP, the vector control cell K562-A:0201-GFP-mCherry vector, and the target cell K562-A:0201-GFP-pp65-mCherry were prepared *in vitro.* CMV-pp65-TCR-T effector cells were co-cultured with target cells or control cells (vector control and blank control) in vitro. After intracellular and extracellular staining, the expression levels of CD137 and IFNy of CMV-pp65-TCR-T cells were detected by flow cytometry.

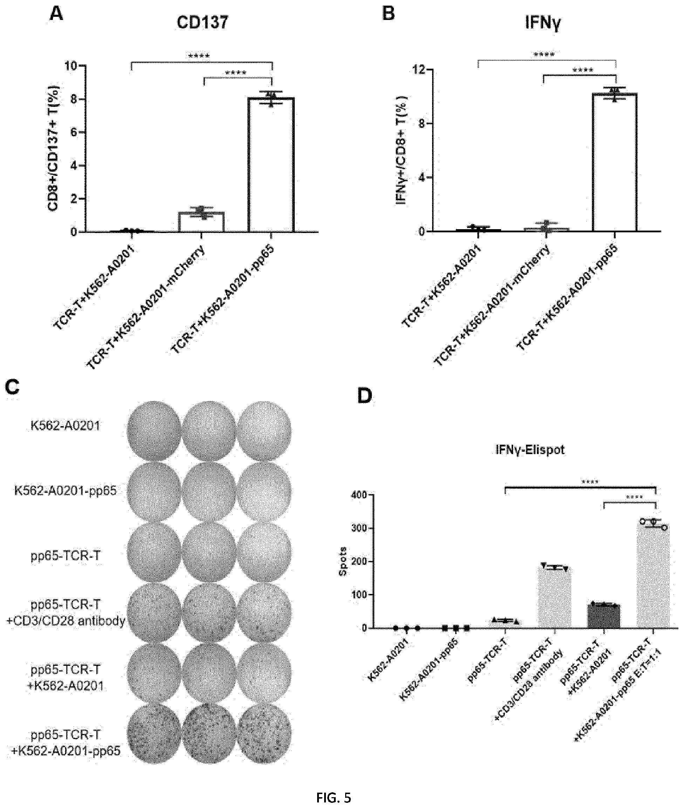

The results of flow cytometry (FIG. 5) showed that K562-A:0201-GFP-pp65-mCherry target cells could specifically activate CMV-pp65-TCR-T cells *in vitro.* After activation, the expression levels of CD137 and IFNγ of CMV-pp65-TCR-T were significantly increased, while the vector control group K562 cells and control K562 cells had no significant activating effect on CMV-pp65-TCR-T cells.

In addition, the IFNγ-Elispot results also showed that K562-A:0201-GFP-pp65-mCherry target cells could specifically activate CMV-pp65-TCR-T cells *in vitro,* and the IFNγ expression level of CMV-pp65-TCR-T was significantly increased after activation, while the control cell K562-A:0201-GFP had no activating effect on CMV-pp65-TCR-T.

### In vitro CMV-pp65-TCR-T killing experiment:

Target cells K562-A:0201-GFP-pp65-mCherry, Reported TCR-T disclosed in CN102656188A and CMV-pp65-TCR-T of this project were prepared in vitro.

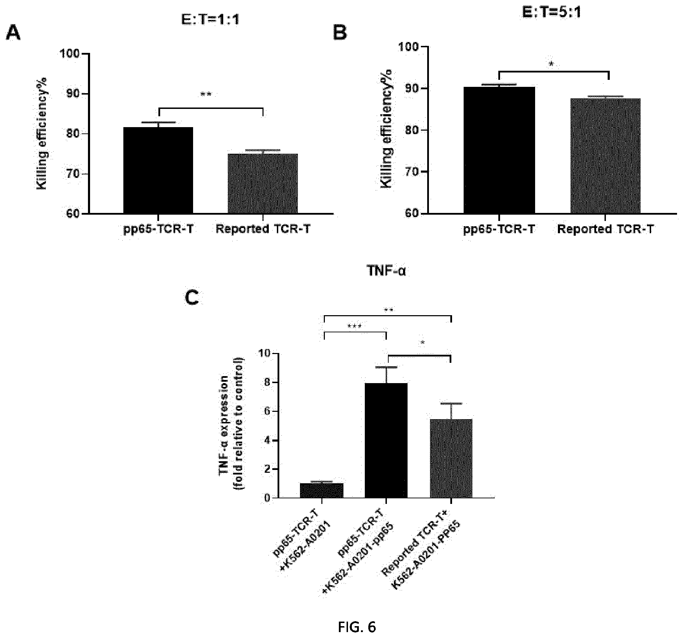

By co-culturing CMV-pp65-TCR-T or Reported TCR-T effector cells with target cells at the effector-target ratio of 1:1 and 5:1 *in vitro* for 24 h, the proportion of GFP+ target cells was detected by flow cytometry. The results of flow cytometry (FIG. 6, A and B) showed that both CMV-pp65-TCR-T and Reported TCR-T cells could kill K562-A:0201-GFP-pp65-mCherry cells expressing pp65 *in vitro,* but the CMV-pp65-TCR-T disclosed in this project was superior to Reported TCR-T in terms of killing effect.

Subsequently, CMV-pp65-TCR-T and Reported TCR-T effector cells were co-cultured with target cells according to the effector-target ratio of 1:1 *in vitro.* After 24h, intracellular staining was performed, and then the expression of cytokine TNF-α was detected by flow cytometry to evaluate the cytokine release of CMV-pp65-TCR-T and Reported TCR-T. The results of flow cytometry showed that CMV-pp65-TCR-T cells screened and identified in this project could release higher levels of TNF-α in vitro than Reported TCR-T, and specifically kill K562-A:0201-GFP-pp65-mCherry cells expressing pp65 (FIG. 6, C), while CMV-pp65-TCR-T did not release TNF-α and IFN-γ cytokines after co-culture with K562-A0201 control cells, indicating its good specificity.

### In vivo CMV-pp65-TCR-T killing experiment-Subcutaneously implanted tumor model:

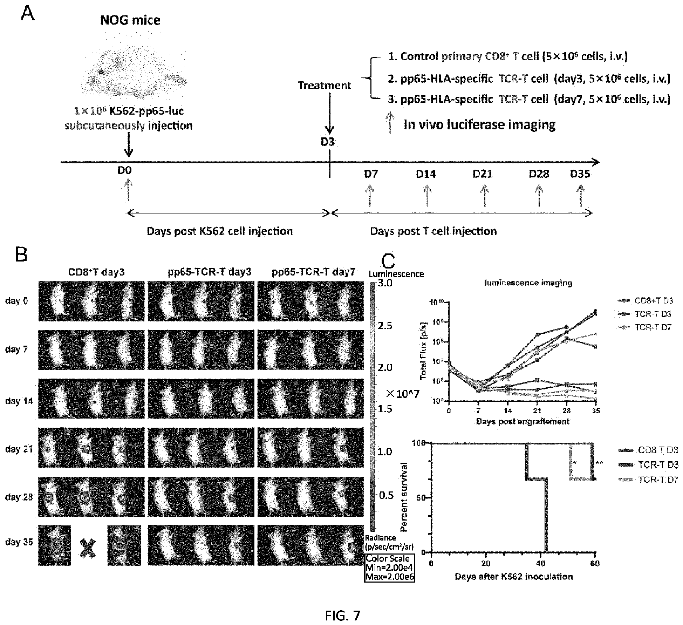

K562-A:0201-GFP-pp65-luciferase target cells and CMV-pp65-TCR-T effector cells were prepared *in vitro.* 1 × 10⁶ target cells were subcutaneously injected into the right flank of NOG mice on Day 0. 5 × 10⁶ primary CD8⁺ T cells (CD8⁺T day3 group) and CMV-pp65-TCR-T cells (pp65-TCR-T day3 group) were injected through the lateral tail vein on Day 3, and 5 × 10⁶ CMV-pp65-TCR-T cells (pp65-TCR-T day7 group) were injected through the lateral tail vein on Day 7 (FIG. 7, A); bioluminescence imaging was performed on mice at 0-5 weeks after tumor cell injection (FIG. 7, B), and the photon number intensity of luciferase in mice was quantified. The results of in vivo experiments showed that the CMV-pp65-TCR-T cells of this project could specifically kill K562-A:0201-GFP-pp65-luciferase target cells *in vivo* (FIG. 7, B and C). Injection of CMV-pp65-TCR-T on day 3 or day 7 could significantly inhibit the growth of tumor cells K562-A:0201-GFP-pp65-luciferase and significantly prolong the survival time of mice (FIG. 7, D). Its killing effect on target cells was significantly better than that of CD8⁺T control cells without pp65-TCR transduction.

### In vivo CMV-pp65-TCR-T killing experiment-metastasis model:

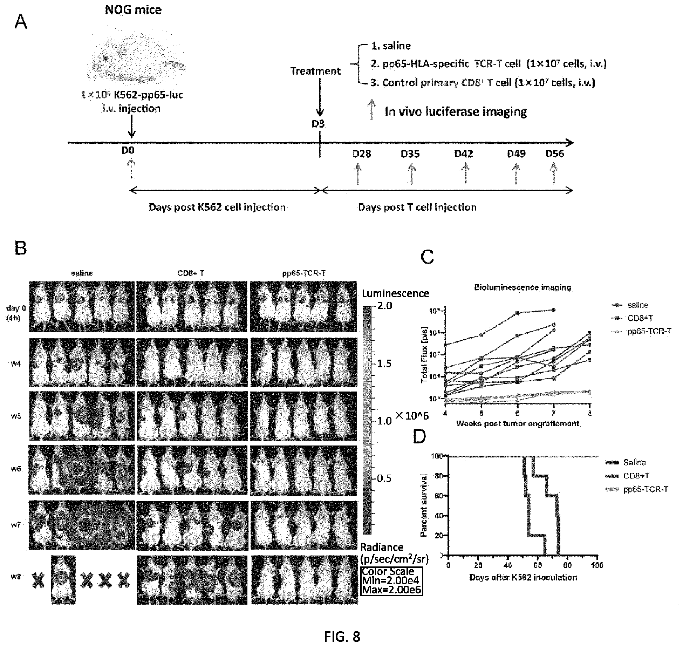

K562-A:0201-GFP-pp65-luciferase target cells and CMV-pp65-TCR-T effector cells were prepared *in vitro.* 1 × 10⁶ target cells were injected into the tail vein of NOG mice on day 0, and 1 × 10⁷ primary CD8⁺ T cells and CMV-pp65-TCR-T cells were injected through the lateral tail vein on day 3 (FIG. 8, A); Bioluminescence imaging detection was performed on mice at 4-8 weeks after tumor cell injection (FIG. 8, B), and the photon number intensity of luciferase in mice was quantified.

The results of in vivo experiments showed that the CMV-pp65-TCR-T cells of this project could specifically kill K562-A:0201-GFP-pp65-luciferase target cells in vivo (FIG. 8, B and C). Injection of CMV-pp65-TCR-T on day 3 could significantly inhibit the growth of tumor cells K562-A:0201-GFP-pp65-luciferase, and its inhibitory effect was significantly better than that of CD8⁺T control cells without pp65-TCR transduction.

### Technial effects

CMV infection is very widespread in the population. The infection rate of Chinese adults is more than 95%, which is usually recessive infection. Most infected people have no clinical symptoms, but under certain conditions, it can invade multiple organs and systems and produce serious disease. CMV activation is common in hematopoietic stem cell transplantation (HSCT) patients. Some patients have poor drug treatment effect or low drug tolerance and cannot complete the whole course of drug treatment. Due to poor immune function, patients can not effectively clear CMV, which can cause a series of related diseases from CMV related fever to organ involvement, with a high mortality. The existing anti-CMV drugs also have many shortcomings, such as single dosage form, similar target, which can lead to granulocyte deficiency /renal damage, etc. Therefore, there is an urgent need for new clinical treatment options for the treatment of CMV infection in HSCT patients. In one aspect, the CMV-TCR-T cells of this project can accurately target and clear the target cells infected with CMV virus; in another aspect, it can also play a long-term protective role through TCR-T cell-mediated immune reconstitution, the thorough clearance of virus and prevention of reinfection indirectly.

The CMV-pp65-TCR-T of this project has the following characteristics:
*In vivo* and *in vitro* experiments showed that CMV-pp65-TCR-T could accurately target and rapidly clear the target cells expressing CMV antigen, have high protein expression in vivo, and no mismatch with the endogenous TCR chain, have strong killing ability and specificity etc. Furthermore, *in vitro* experiments showed that CMV-pp65-TCR-T of this project has more significant killing effect on target cells than Reported-TCR-T disclosed in CN102656188A and can be effectively applied to the treatment of CMV infection in HSCT patients or other transplant patients.

In addition, compared with the existing related published inventions, this project provides the *in vitro* experimental evidence of ELISPOT (enzyme-linked immunospot), which is the recognized gold standard in the field of TCR-T, indicating that the TCR screened in this project can specifically recognize CMV antigen; at the same time, the invention of this project not only uses the tail vein model to confirm that the T cells loaded with CMV specific TCR can specifically clear the target cells expressing CMV antigen, but also uses the subcutaneous tumorigenesis model that is closer to the clinical CMV infection situation, and also confirms that the T cells loaded with CMV specific TCR obtained by screening in this project can specifically clear the target cells expressing CMV antigen.

In general, the invention of this project provides complete *in vitro* and *in vivo* data that meet the field standards and are closer to the clinical CMV infection situation, fully confirming the specificity and effectiveness of the CMV antigen-specific TCR obtained by screening.

All documents mentioned in the present invention are cited as references in the present application, just as each document is cited separately as a reference. In addition, it should be understood that after reading the above teaching content of the invention, those skilled in the art can make various changes or modifications to the invention, and these equivalent forms also fall within the scope of the claims attached to the application.

## Claims

1. A T cell receptor (TCR), wherein the T cell receptor comprises a TCR α chain variable domain and a TCR β chain variable domain, and the amino acid sequence of CDR3 of the TCR α chain variable domain is CAFPYNNNDMRF (SEQ ID No. 13); and/or
the amino acid sequence of CDR3 of the TCR β chain variable domain is CASSLEGYTEAFF (SEQ ID No. 21).

2. The T cell receptor (TCR) according to claim 1, wherein the three complementarity determining regions (CDRs) of the TCR α chain variable domain are:
α-CDR1: SSNFYA (SEQ ID NO.9),
α-CDR2: MTLNGDE (SEQ ID NO.11),
α-CDR3: CAFPYNNNDMRF (SEQ ID NO.13); and/or
the three complementarity determining regions of the TCR β chain variable domain are:
β-CDR 1: MNHEY (SEQ ID NO.17),
β-CDR 2: SMNVEV (SEQ ID NO.19),
β-CDR 3: CASSLEGYTEAFF (SEQ ID NO.21).

3. The TCR according to claim 1, wherein it comprises a TCR α chain variable domain and a TCR β chain variable domain, and the TCR α chain variable domain is an amino acid sequence having at least 90% sequence identity with SEQ ID No.7; and/or, the TCR β chain variable domain is an amino acid sequence having at least 90% sequence identity with SEQ ID No.15.

4. The TCR according to claim 1, wherein the amino acid sequence of the TCR is shown in SEQ ID No.1.

5. A multivalent TCR complex, wherein it comprises at least two TCR molecules, and at least one of the TCR molecules is the TCR according to any one of the previous claims.

6. A nucleic acid molecule, wherein the nucleic acid molecule comprises a nucleic acid sequence encoding the TCR molecule according to claim 1 or a complementary sequence thereof.

7. The nucleic acid molecule according to claim 6, wherein the nucleic acid molecule comprises a nucleotide sequence of SEQ ID No.8, which encodes the TCR α chain variable domain; and/or
the nucleic acid molecule comprises a nucleotide sequence of SEQ ID No.16 which encodes the TCR β chain variable domain.

8. A vector, wherein the vector contains the nucleic acid molecule according to claim 6; preferably, the vector is a viral vector; more preferably, the vector is a lentiviral vector.

9. An isolated host cell, wherein the host cell contains the vector according to claim 8 or has the exogenous nucleic acid molecule according to claim 6 integrated in chromosome.

10. A cell, wherein the cell is transduced with the nucleic acid molecule according to claim 6 or the vector according to claim 8; preferably, the cell is a T cell or stem cell.

11. A pharmaceutical composition, wherein the composition contains a pharmaceutically acceptable carrier, and the TCR according to any one of claims 1-4, the TCR complex according to claim 5, the nucleic acid molecule according to claim 6, or the cell according to claim 10.

12. Use of the T cell receptor according to any one of claims 1-4, or the TCR complex according to claim 5, or the cell according to claim 10 in the manufacture of a drug for treating cytomegalovirus infection.

13. A method for treating diseases, wherein it comprises administering an appropriate amount of the TCR according to any one of claims 1-4, the TCR complex according to claim 5, the nucleic acid molecule according to claim 6, or the cell according to claim 10 or the pharmaceutical composition according to claim 11 to a subject in need of treatment.

14. The method according to claim 13, wherein the disease is a cytomegalovirus infection related disease (cytomegalovirus infection), such as CMV retinitis, CMV pneumonia, CMV gastroenteritis, CMV encephalitis, etc.
